# FASCICULE DE BREVET EUROPEEN

(11) **EP 1 420 752 B1**
(45) Date de publication et mention de la délivrance du brevet: **22.02.2012**
(21) Numéro de dépôt: 02796297.6
(22) Date de dépôt: 20.08.2002
(51) Int. Cl.: A61Q 5/10, A61K 8/68

(54) **CREME COLORANTE CAPILLAIRE**
HAARFÄRBECREME
DYEING HAIR CREAM

(30) Priorité: 28.08.2001 FR 0111156
(43) Date de publication de la demande: 26.05.2004
(73) Titulaire: FAUVERT (SA), 13593 Aix-En-Provence Cedex 03 (FR)
(72) Inventeur: THOMAS, Brigitte, F-13008 Marseille (FR)
(74) Mandataire: Roman, Michel
(86) Numéro de dépôt international: PCT/FR2002/002904
(87) Numéro de publication internationale: WO 2003/017957

(56) Documents cités:
- EP-A- 0 673 641
- DE-A- 19 735 852
- FR-A- 2 738 483

## Description

La présente invention concerne une crème colorante capillaire.

Elle a pour objet l'association synergique de polymères substantifs et de palmitamido-serinate de myristyle et d'extraits d'acides aminés végétaux quaternisés, combinée à une crème spécifique de coloration oxydative permanente des cheveux. Cette association trouvera également des applications dans les domaines des shampooings, crèmes et masques de soins, mousses et laques aérosols, produits pour l'ondulation, ou la fixation des cheveux et tout autre produit de coloration ou non.

Cette invention se rapporte d'une manière générale au secteur industriel et commercial de la fabrication et de la diffusion de produits cosmétiques destinés en particulier aux professionnels du traitement des cheveux et a pour objet de permettre une coloration durable des cheveux dans toutes les nuances répertoriées, quelle que soit la nature et l'état initial de la chevelure à traiter.

Pendant des siècles, la coloration des cheveux n'a représenté qu'une mode réservée à quelques privilégiés. Il y a quelques dizaines d'années, elle n'était qu'une "nécessité" : cacher les cheveux blancs pour' paraître plus jeune. Aujourd'hui, le nombre de femmes, et aussi d'hommes, qui modifient la couleur de leur chevelure pour lui conférer un charme nouveau est devenu considérable. Ce nombre s'accroît sans cesse et les motivations sont multiples: cacher les cheveux blancs, les éclaircir, ajouter des reflets, déjaunir ou embellir les cheveux gris, suivre la mode ...

De nombreuses catégories sont à distinguer dans la coloration: le simple rinçage, le reflet, la teinture, le renforçateur de coloration, la coloration progressive, la coloration temporaire, la coloration directe et la coloration permanente.

Les produits de coloration permanente sont de très loin les plus employés. Ils apportent une modification sensible et permanente de la couleur naturelle. Le marché actuel de la coloration d'oxydation, au niveau des professionnels de la coiffure, représente entre 40 et 50% du chiffre d'affaire des salons de coiffure.

On peut distinguer deux types de formulations pour la coloration permanente :
- la coloration, dite « ton sur ton », qui n'apporte pas de réel pouvoir d'éclaircissement car n'employant pas d'ammoniaque mais respectant mieux, de par ce fait, la fibre kératinique.
- la coloration d'oxydation éclaircissante, qui peut éclaircir de manière très sensible la couleur naturelle des cheveux. En fait, durant l'application, il y a éclaircissement grâce à un oxydant et au processus de coloration.

La présente invention porte sur ce type de coloration.

Les colorants employés, dits "d'oxydation", sont les seuls qui puissent donner aux cheveux des colorations permanentes dans une infinité de tons tout en couvrant parfaitement les cheveux blancs. Ils doivent être mélangés avec un produit oxydant au moment de l'emploi.

Le cheveu est une structure kératinique morte formant une fibre composée de nombreuses fibrilles. Sa surface est constituée par la cuticule, couche protectrice formée de cellules aplaties recouvrant partiellement la cellule sous-jacente à la manière des tuiles d'un toit en formant des écailles se chevauchant. Tout au long de la fibre capillaire, on rencontre les pigments mélaniques responsables de la coloration naturelle du cheveu.

La structure du cheveu, dont le pH est normalement compris entre 6 et 6,5, présente des propriétés particulières : à pH acide, les écailles sont fermées et lisses, la structure du cheveu est alors bien protégée, alors qu'à pH basique, les écailles s'ouvrent, permettant ainsi un accès à la structure interne et donc la possibilité d'effectuer des actions chimiques à l'intérieur de la cuticule.

Pour assurer une coloration durable, il faut pouvoir atteindre les pigments de mélanine contenus à l'intérieur du cheveu et, dans ce but, provoquer l'écartement des écailles. Ce résultat est obtenu au moyen d'un agent alcalinisant.

Actuellement, l'ammoniaque est l'agent alcalinisant le plus couramment employé en coloration d'oxydation car il présente l'avantage de participer activement au processus de décoloration, simultanément à celui d'oxydation des précurseurs de colorant pour former in situ des colorants capillaires. Néanmoins, l'ammoniaque est un produit fortement irritant, susceptible de causer des lésions et des irritations cutanées, aussi bien aux professionnels qui appliquent les produits qu'aux clients des salons de coiffures.

La plupart des produits colorants existants, lorsqu'ils sont utilisés de façon répétitive, provoquent à la longue un décollement des écailles rendant les cheveux fourchus et ternes.

Les teintes se présentent souvent dans des gammes limitées ou la couleur obtenue est différente selon la teinte initiale du cheveu ou son état.

La présente invention a pour but de remédier à ces inconvénients en offrant un affinement et un arrangement des écailles du cheveu ayant un effet restructurant et lissant améliorant la structure et l'aspect du cheveu, dans le processus d'une crème colorante permettant l'obtention de teintes stables et fidèles et une bonne couverture des cheveux blancs.

Le document FR-A-2738483 enseigne que des alkoyles serinates d'alkyl, en combinaison avec certains polyacrylates et polyuréthanes confèrent aux cheveux rémanence, gainage, restauration.

Elle est constituée d'un complexe associatif, agent de soin, à base de palmitamido séranite de myristyle, de polymères substantifs et d'extraits d'acides aminés végétaux quaternisés combinée à un support crème spécifique adapté à la la coloration d'oxydation, comportant un agent alcalinisant et un système colorant d'oxydation permettant d'obtenir toutes les nuances en combinant les compositions tinctoriales contenant des précurseurs de colorants d'oxydation associés ou non aux coupleurs classiques, la teinte finale étant révélée par l'action d'un produit oxydant.

La description détaillée ci-après se rapporte à un exemple non limitatif d'une des formes de réalisation de l'objet de l'invention.

La crème colorante capillaire selon la présente demande est constituée de trois sortes d'éléments étudiés pour obtenir un résultat optimal en étant combinés :
- un support crème comportant le complexe associatif
- un alcalinisant.
- un groupe de colorants d'oxydation (coupleurs et bases) dont le dosage correct permet d'obtenir tous les tons et nuances désirés.

Le support crème proprement dit est constitué du mélange de trois groupes de composés dont les proportions en poids sont les suivantes
Groupe A :
   - 20 à 60% d'eau
   - 10 à 40% de cetearyl alcohol
   - 1 à 20% de ceteareth-25
   - 0,1 à 10% de ceteth-2
Groupe B :
   - 2 à 20% de peg-2 Rapeamine
   - 0,1 à 10% de peg-4 Rapeseedamide
   - 0,1 à 10% de propylène glycol
Groupe C - Complexe Associatif
   - 0,01 à 10% d'hydroxypropyltrimonium (protéine de blé hydrolysée)
   - 0,001 à 12% de polyquaternium-22
   - 0,001 à 12% de polyquaternium-7
   - 0,001 à 12% de palmitamido-sérinate de myristyle
   Et préférentiellement:
   - 0,1 à 1% d'hydroxypropyltrimonium (protéine de blé hydrolysée)
   - 0,02 à 6% de polyquaternium-22
   - 0,02 à 6% de polyquaternium-7
   - 0,02 à 6% de palmitamido-sérinate de myristyle

Le palmitamido-sérinate de myristyle est un analogue chimique des céramides 2. Il agit pour le renforcement de la structure et du gainage du cheveu et protège les cheveux décolorés et permanentés. Il permet de protéger de façon très efficace des cheveux ayant subi des dommages oxydatifs importants, tels qu'une décoloration. Grâce à son caractère hydrophobe, le palmitamido-sérinate de myristyle est incorporé naturellement entre les cellules cuticulaires et permet, par sa très grande affinité avec les protéines environnantes, d'exercer son rôle de " ciment" et, ainsi, d'augmenter la cohésion cellulaire. L'utilisation du palmitamido-sérinate de myristyle permet un aplanissement, un affinement et un arrangement plus régulier des écailles du cheveu, qui sont moins décollées, reflétant un effet restructurant et lissant, ainsi qu'un renforcement net de la structure du cheveu.

Cette molécule est très proche, des acyl-sphinganines (céramide 2 naturel) trouvés sur les cheveux.

L'alcalinisant pourra être de l'ammoniaque ou une base telle que la monoéthanolamine (MEA), la triéthanolamine (TEA) ou le bicarbonate de sodium (BS ou autre hydroxyde métallique.

Les colorants d'oxydation (bases + couleurs) sont ceux utilisés couramment dans la constitution des crèmes ou gels oxydatifs de coloration.

Le positionnement des divers éléments constitutifs donne à l'objet de l'invention un maximum d'effets utiles qui n'avaient pas été, à ce jour, obtenus par des produits et procédés similaires.

## Revendications

1. Crème colorante capillaire, destinée en particulier aux professionnels du traitement des cheveux et ayant pour objet de permettre une coloration durable des cheveux dans toutes les nuances répertoriées, quelle que soit la nature et l'état initial de la chevelure à traiter,
**caractérisée par** le complexe association, agent de soin, à base de palmitamido-sérinate de myristyle, de polymères substantifs (polyquaternium-22 et polyquaternium-7) et d'extraits d'acides aminés végétaux quaternisés (hydroxypropyltrimonium Hydrolysed Wheat Protein), combiné à un support crème spécifique adapté à la coloration d'oxydation, comportant un agent alcalinisant et un système colorant d'oxydation permettant d'obtenir toutes les nuances en combinant les compositions tinctoriales contenant des précurseurs de colorants d'oxydation associés ou non aux coupleurs classiques, la teinte finale étant révélée par l'action d'un produit oxydant.

2. Crème colorante capillaire selon la revendication 1, **se caractérisant par le fait que** l'agent de soin incorporé est un complexe associatif d'un produit analogue chimique des céramides 2, le palmitamido-sérinate de myristyle avec des polymères substantifs et des acides aminés d'origine végétale.

3. Crème colorante capillaire selon la revendication 2, **se caractérisant par le fait que** le support crème est composé du mélange de trois groupes de composants dont le dosage en poids est le suivant :
Groupe A
- 20 à 60% d'eau
- 10 à 40% de cetearyl alcohol
- 1 à 20% de ceteareth-25
- 0,1 à 10% de ceteth-2
Groupe B
- 2 à 20% de peg-2 Rapeamine
- 0,1 à 10% de peg-4 Rapeseedamide
- 0,1 à 10% de propylène glycol
Groupe C
- 0,01 à 10% d'hydroxypropyltrimonium (protéine de blé hydrolysée)
- 0,001 à 12% de polyquaternium-22
- 0,001 à 12% de polyquaternium-7
- 0,001 à 12% de palmitamido-sérinate de myristyle
de préférence:
- 0,1 à 1 % d'hydroxypropyltrimonium (protéine de blé hydrolysée)
- 0,02 à 6% de polyquaternium-22
- 0,02 à 6% de polyquaternium-7
- 0,02 à 6% de palmitamido-sérinate de myristyle

4. Crème colorante capillaire selon la revendication 3, **se caractérisant par le fait que** l'agent alcalinisant est constitué d'une basé telle que l'ammoniaque, la monoéthanolamine (MEA), la triéthanolamine (TEA), le bicarbonate de sodium (BS) ou l'hydroxyde de sodium, ou autre hydroxyde métallique.

5. Crème colorante capillaire selon la revendication 4, **se caractérisant par** l'emploi des classiques colorants et précurseurs de colorants d'oxydation couramment utilisés.

## Claims

1. Dyeing hair cream, intended in particular for use by hair treatment professionals, for the purpose of durable dyeing of the hair in all the shades, whatever the nature and
the initial condition of the hair to be treated,
**characterized by** the complex association of care agent containing of myristyle palmitamido-serinate, substantive polymers (polyquaternium-22 and polyquaternium-7) and extracts of quaternized amino-acids (hydroxypropyl- trimonium - hydrolyzed wheat protein), combined with a specific cream- medium suitable for oxidation dyeing, comprising an alkalizing agent and an oxidation dye enabling all shades to be obtained by combining the tinctorial compositions containing precursors of oxidation dyes whether or not associated with standard couplers, the final color being obtained by the action of an oxidizing product.

2. Dyeing hair cream according to claim 1, **characterized in that** the hair care product is an associative complex of a chemical product similar to ceramide 2, myristyle palmitamido-serinate with substantive polymers and amino-acids of plant origin.

3. Dyeing hair cream according to claim 2, **characterized in that** the cream medium is composed of the mixture of three groups of components whose dosage by weight is as follows:
Group A
- 20 to 60% water
- 10 to 40% of cetearyl alcohol
- 1 to 20% of ceteareth-25
- 0.1 to 10% of ceteth2
Group B
- 2 to 20% of peg-2 Rapeamine
- 0.1 to 10% of peg-4 Rapeseedamide
- 0.1 to 10% of propylene glycol
Group C
- 0.01 to 10% of hydroxypropyltrimonium (hydrolized wheat protein)
- 0.001 to 12% of polyquaternium-22
- 0.001 to 12% of polyquaternium-7
- 0.001 to 12% of myristyle palmitamido-serinate
preferably
- 0.1 to 1% of hydroxypropyltrimonium (hydrolised wheat protein)
- 0.02 to 6% of polyquaternium-22
- 0.02 to 6% of polyquaternium-7
- 0.02 to 6% of myristyle palmitamido-serinate

4. Dyeing hair cream according to claim 3, **characterized in that** the alkalizing product consists of a basic such as ammonia, monoethanolamine (MEA), triethanolamine (TEA), sodium bicarbonate (BS) or sodium hydroxide, or another metal hydroxide.

5. Dyeing hair cream according to claim 4, **characterized by** the use of traditional dyes and precursors of oxidation dyes generally used.

## Patentansprüche

1. Haarfärbecreme mit Kapillarwirkung, insbesondere bestimmt für Fachleute der Haarbehandlung, zum Zweck der dauerhaften Haarfärbung in allen verzeichneten Farbnuancen, unabhängig von der Art und dem Ausgangszustand des zu behandelnden Haares,
**gekennzeichnet durch** den Komplex gebildet aus Pflegemittel auf Palmitamido-Myristyl-Serinate-Basis und substantiven Polymeren (Polyquaternium-22 und Polyquaternium-7) sowie Auszügen aus quaternisierten, pflanzlichen Aminosäuren (4-Hydroxybenzoesäuremethylester Hydrolysed Wheat Protein),
kombiniert mit einer Cremeunterlage, die besonders für die Oxidationsfärbung geeignet ist und einen alkalinisierenden Wirkstoff und ein Oxidationsfärbesystem enthält, mit dem durch Mischen der Farbtonkompositionen alle Farbnuancen gebildet werden können, die Vorläufer von Oxidationsfarbstoffen enthält, die an herkömmliche Koppler gebunden sind, wobei der endgültige Farbton **durch** die Wirkung eines Oxidationsmittels entwickelt wird

2. Haarfärbecreme mit Kapillarwirkung gemäß Patentanspruch 1, **gekennzeichnet dadurch, dass** das enthaltene Pflegemittel ein Komplex ist, gebildet aus einem chemisch den Ceramiden-2 verwandten Produkt, dem Palmitamido-Myristyl-Serinate mit substantiven Polymeren und pflanzlichen Aminosäuren ist.

3. Haarfärbecreme mit Kapillarwirkung gemäß Patentanspruch 2, **gekennzeichnet dadurch, dass** die Cremeunterlage eine Mischung aus drei Komponentengruppen in folgenden Gewichtsanteilen ist:
Gruppe A
- 20 bis 60% Wasser
- 10 bis 40% Cetylstearylalkohol
- 1 bis 20% Ceteareth-25
- 0,1 bis 10% Ceteth-2
Gruppe B
- 2 bis 20% Peg-2 Rapeamine
- 0,1 bis 10% Peg-4 Rapeseedamide
- 0,1 bis 10% de Propylenglycol
Gruppe C
- 0,01 bis 10% 4-Hydroxybenzoesäuremethylester (hydrolysiertes Weizenprotein)
- 0,001 bis 12% Polyquaternium-22
- 0,001 bis 12% Polyquaternium-7
- 0,001 bis 12% Palmitamido-Myristyl-Serinate
vorzugsweise:
- 0,1 bis 1 % 4-Hydroxybenzoesäuremethylester (hydrolysiertes Weizenprotein)
- 0,02 bis 6% Polyquaternium-22
- 0,02 bis 6% Polyquaternium-7
- 0,02 bis 6% Palmitamido-Myristyl-Serinate

4. Haarfärbecreme mit Kapillarwirkung gemäß Patentanspruch 3, **gekennzeichnet dadurch, dass** der alkalinisierende Wirkstoffs eine Basis aus Ammoniak, Monoethanolamin (MEA), Triethanolamin (TEA), Natriumbicarbonat (BS), Natriumhydroxid oder einem anderen Metallhydroxid besitzt.

5. Haarfärbecreme mit Kapillarwirkung gemäß Patenanspruch 4, **gekennzeichnet durch** Verwendung herkömmlicher Farbstoffe und Vorläufern häufig verwendeter OxidationsFarbstoffe.
